# EUROPEAN PATENT APPLICATION

(11) **EP 4 035 667 A1**
(43) Date of publication of application: **03.08.2022**
(21) Application number: 22163870.3
(22) Date of filing: 21.04.2017
(51) Int. Cl.: A61K 31/353, A61K 31/352, A61K 31/382, A61K 31/473, A61K 47/44, A61K 47/46, A61K 9/00, A61K 9/02, A61P 35/00, A61K 31/282, A61K 31/337, A61K 31/555

(54) **CHEMOTHERAPY IMPROVEMENTS WITH IDRONOXIL**

(30) Priority: 22.04.2016 US 201662326144 P
(62) Divisional of application: 17785173.0
(71) Applicant: Noxopharm Limited, Gordon, New South Wales 2072 (AU)
(72) Inventor: KELLY, Graham, Gordon, 2072 (AU)
(74) Representative: Carpmaels & Ransford LLP

(57) **Abstract**

A first composition including a platin, a taxane, or combination of same and a second composition including an isoflavonoid which is idronoxil, for use in a method for treating or preventing cancer.

## Description

### Field of the invention

The invention relates to cancer chemotherapy, particularly to therapy of cancers having drug-resistance or insensitivity to chemotherapy, particularly including drugs based on a platinum structure and drugs based on a taxane structure, or combination chemotherapy involving platinums and taxanes.

### Background of the invention

Drugs that inflict lethal and sub-lethal damage on cells with the purpose of inhibiting cell division (cytostasis) or cell death (cytotoxicity) are widely used in medicine in the treatment of conditions characterised by abnormal proliferation of cells. This includes benign and malignant neoplasias, dysplasias and metaplasias, as well as non-cancerous conditions such as rheumatoid arthritis.

These drugs generally are referred to collectively as cytotoxic chemotherapies. One basis of categorising them is their general chemical structure.

One example of this class of drug is a family of related compounds based on complexes of platinum. They are referred to informally as platins and include such commercial entities as cisplatin, carboplatin, oxaliplatin and nedaplatin.

The platins are active against proliferating cells by binding to their DNA and interfering with its repair mechanism, eventually leading to cell death. It is thought that the first step in the cellular process is that a molecule of water replaces one of the chloride ions of the platin molecule. The resulting intermediate structure can then bind to a single nitrogen on a DNA nucleotide. Following that, the second chloride is also replaced by another water molecule and the platinum agent then binds to a second nucleotide. Binding studies of cisplatin with DNA have indicated a preference for nitrogen 7 on two adjacent guanines on the same strand. It also binds to adenine and across strands to a lesser extent.

The binding of cisplatin to DNA causes production of intrastrand cross-links and formation of DNA adducts. The adducts or drug-DNA complexes attract the attention of DNA repair proteins which become irreversibly bound. The resulting distortion to the shape of the DNA by the binding of the platin drug prevents effective repair and hence, cell death.

Another example of the cytotoxic chemotherapies is a family of drugs referred to as taxanes because they originally were identified from plants of the genus Taxus (yews) and feature a taxadiene core. Commercial examples of taxanes include paclitaxel, docetaxel and cabazitaxel.

The principal mechanism of action of the taxane class of drugs is disruption of the cell's microtubules. Microtubules are essential to cell division by providing the mitotic spindle on which chromosomes assemble prior to cell division. The taxanes stabilise the guanosine diphosphate-bound tubulin in the microtubule, thereby inhibiting the process of cell division as depolymerisation is prevented.

While each of these two classes of cytotoxic drugs are approved for use as monotherapies, that is, to be used on their own, it is common for both classes to be administered in combination with the rationale that a combination of both mechanisms of action will deliver a more beneficial anti-cancer effect than either mechanism alone.

These two classes of cytotoxic drugs represent between them the two most commonly used therapies in the management of cancers, particularly solid cancers.

An inherent problem with all platin and taxane based chemotherapies is their non-selectivity. That is, they do not distinguish between a cancer cell and a non-cancer cell. Their molecular target in each case is common to both healthy and cancerous cells. Their therapeutic use relies on the agent's cytotoxic effect being directly proportional to the cell's rate of metabolic activity and growth, which in cancer patients often is the cancer tissue.

However, all tissues in the body undergo cell division to a greater or lesser extent, which exposes these tissues also to the cytotoxic effect of platin and taxane based chemotherapies. Tissues most at risk are the lining of the gut, bone marrow, hair follicles, testicles and nerve tissue. Symptoms include anemia, leucopaenia, nausea, vomiting, diarrhoea, mucositis, hair loss, loss of hearing and peripheral nerve damage.

This inherent non-selective nature of these agents has four unfortunate consequences.

First, it serves to restrict the amount of drug that can be given to patients, in terms of the amount of drug at any one time as well as the duration of treatment. This has the effect of meaning that in the great majority of cases, the treatment is unlikely to be curative. The evidence of this lies in the fact that 10-year survival prospects for most of the common forms of cancer, as well as for many of the less common forms, have shown little or no improvement over the past 40 years despite platin and taxane based chemotherapies remaining the most commonly-used form of cancer therapy. For cancers of the mouth, oesophagus, stomach, large bowel, kidney, bladder, pancreas, brain, ovary and cervix, survival prospects remain remarkably poor.

Second, it serves to limit the ability to use platin and taxane based chemotherapies in patients unlikely to be able to tolerate the rigours of platin and taxane based chemotherapies associated with even standard dosages. This refers particularly to elderly or frail individuals, or individuals with underlying diseases unrelated to to the cancer, such as cardiovascular disease.

Third, it exposes young patients (infants, children, adolescents) to physical damages that can be permanent and result in serious lifelong consequences.

Fourth, it prevents the level of dosage being elevated to overcome the development of drug-resistance mechanisms by cancer cells. Such mechanisms have been the subject of much study and remain largely obscure, although a variety of different biological responses have been described including up-regulation of pumping mechanisms that serve to expel drugs from the cell, up-regulation of proteins that block the entry of drugs into the cell, up-regulation of repair mechanisms such as DNA repair mechanisms, and the up-regulation of signalling pathways as alternatives to targeted pathways. Such drug-resistance mechanisms occur either inherently (known as primary resistance) or develop following exposure to platin and taxane based chemotherapies (known as acquired resistance). The mechanisms involved in primary and acquired resistance are thought to be similar. Resistant against one form of cytotoxic drug in general confers resistance on other forms of cytotoxic drug irrespective of their mechanism of cytotoxic action. This is known as multi-drug resistance.

Accordingly there is a strong need to identify new, improved, better and/or alternative treatment regimes involving cytotoxic chemotherapy based on platins and/or taxanes, for the treatment of cancer and diseases associated with undesirable proliferation of cells.

There is a further need for methods utilising agents that address undesirable health effects associated with the use of platin and/or taxane therapy.

There is a further need to ameliorate the undesirable side-effects of platin and/or taxane therapy that would enable such therapy to be administered at effective levels to individuals such as the elderly, frail and chronically ill, who otherwise would not be able to tolerate such therapy.

There is a further need to be able to deliver cytotoxic agents in such a way as to overcome the presence of drug-resistance mechanisms to platins and/or the taxanes.

There is also a need to minimise the dosage or amount of platin and/or taxane required for therapeutic effect.

Reference to any prior art in the specification is not an acknowledgment or suggestion that this prior art forms part of the common general knowledge in any jurisdiction or that this prior art could reasonably be expected to be understood, regarded as relevant, and/or combined with other pieces of prior art by a skilled person in the art.

### Summary of the invention

The invention seeks to address one or more of the above discussed needs or limitations and in one embodiment provides a method for treating or preventing cancer. The method includes administering to a person in need thereof:
- a first composition including:
   a platin; or
   a taxane; or
   a combination of a platin and a taxane;
- a second composition including:
   a compound of general formula (I) : 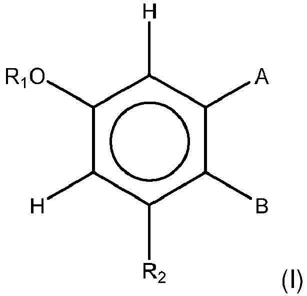 wherein
   R₁ is H, or R_{A}CO where R_{A} is C₁₋₁₀ alkyl or an amino acid;
   R₂ is H, OH, or R_{B} where R_{B} is an amino acid or COR_{A} where R_{A} is as previously defined;
   A and B together with the atoms between them form a six membered ring selected from the group 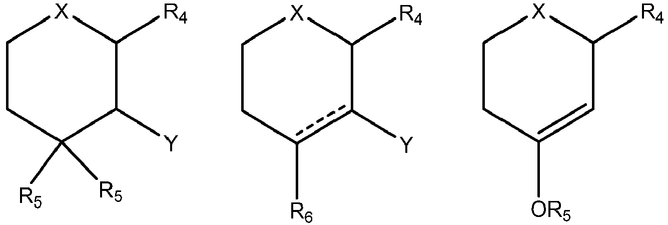 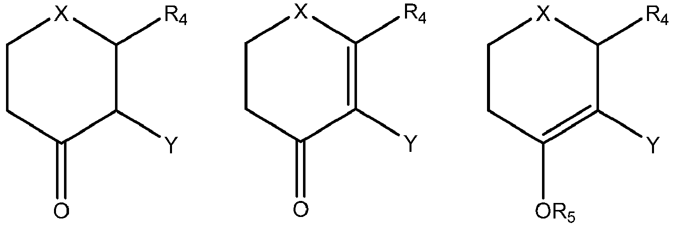 wherein
   R₄ is H, COR_{D} where R_{D} is H, OH, C₁-₁₀ alkyl or an amino acid, CO₂R_{C} where R_{C} is C₁-₁₀ alkyl, COR_{E} where R_{E} is H, C₁-₁₀ alkyl or an amino acid, COOH, COR_{C} where R_{C} is as previously defined, or CONHR_{E} where R_{E} is as previously defined;
   R₅ is H, CO₂R_{C} where R_{C} is as previously defined, or COR_{C}OR_{E} where R_{C} and R_{E} are as previously defined, and where the two R₅ groups are attached to the same group they are the same or different;
   R₆ is H, CO₂R_{C} where R_{C} is as previously defined, COR_{C}OR_{E} where R_{C} and R_{E} are as previously defined, substituted or unsubstituted aryl or substituted or unsubstituted heteroaryl;
   X is O, N or S;
   Y is 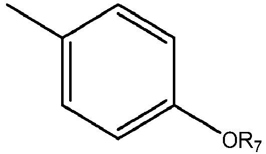 where R₇ is H, or C₁₋₁₀ alkyl; and
   " 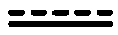 " represents either a single bond or a double bond;
   wherein the second composition is administered to the rectum, vagina or urethra of the person.

The method may utilise a sub-optimal or sub-therapeutic dose of a platin, a taxane, or combination of same.

The method may be applicable to the treatment of cancer cells that are not treated by a platin alone, or by a taxane alone, or by a combination of platin and taxane alone.

In another aspect the method may be applicable to the treatment of cancer cells that have acquired resistance to therapy with a platin alone, or a taxane alone, or a combination of platin and taxane alone.

In one embodiment the first and second compositions are administered according to a treatment cycle in which:
- the first composition is administered once in a treatment cycle, the first composition being first administered after the second composition is first administered;
- the second composition is administered for at least a period of the treatment cycle.

A treatment cycle commences on the day of administration of the first composition and is completed on the day prior to when a next administration of the first composition would become due. Thus, a first treatment cycle commences on the day of first administration of the first composition and is completed on the day prior to when the second administration of the first composition would become due.

The method may include more than one treatment cycle.

The method may include less than 6 treatment cycles, preferably 2 to 4 treatment cycles.

A treatment cycle according to the method may be greater than 21 to 28 days, preferably 30, 60 or 90 days.

In a further embodiment, the second composition may be administered in the absence of administration of the first composition for a period of time prior to the first treatment cycle.

In the above described methods, the taxane may be paclitaxel and the platin may be carboplatin.

In another embodiment there is provided a compound of general formula (I) as described herein for use in treating or preventing cancer in a person, wherein the compound is provided to the rectum, vagina or urethra of the person, and wherein the person has received, or is to receive a platin or a taxane or a combination of a platin and a taxane. In one embodiment, the person has received, or is to receive a sub-optimal dose of a platin or a taxane or a combination of a platin and a taxane. The cancer cells of the person may be cells that are not treated by a platin alone, or by a taxane alone, or by a combination of platin and taxane alone, for example the cells may have developed resistance to taxane or platin therapy.

The platin or taxane or combination of platin and taxane may be provided in the form of a first composition. The compound of general formula (I) may be provided in the form of a second composition.

In one embodiment the compound of general formula (I) is for use in a person who has not yet received, and who is to receive the first composition. In another embodiment, the compound of general formula (I) is for use in a person who has received the first composition.

In one embodiment the first and second compositions are for use according to a treatment cycle in which:
- the first composition is administered once in a treatment cycle, the first composition being first administered after the second composition is first administered;
- the second composition is administered for at least a period of the treatment cycle.

A treatment cycle commences on the day of administration of the first composition and is completed on the day prior to when a next administration of the first composition would become due. Thus, a first treatment cycle commences on the day of first administration of the first composition and is completed on the day prior to when the second administration of the first composition would become due. The compound of general formula (I) may be for use in a person who has received more than one treatment cycle or less than 6 treatment cycles, preferably 2 to 4 treatment cycles. The compound of general formula (I) may be for use in a treatment cycle that is greater than 21 to 28 days, preferably 30, 60 or 90 days.

The compound of general formula (I) may be for use in the absence of administration of the first composition for a period of time prior to the first treatment cycle.

Preferably the taxane may be paclitaxel and the platin may be carboplatin.

In another embodiment there is provided a use of a compound of general formula (I) as described herein for treating or preventing cancer in a person, wherein the compound is provided to the rectum, vagina or urethra of the person, and wherein the person has received, or is to receive a platin or a taxane or a combination of a platin and a taxane. In one embodiment the person has received, or is to receive a sub-optimal dose of a platin or a taxane or a combination of a platin and a taxane. The cancer cells of the person may be cells that are not treated by a platin alone, or by a taxane alone, or by a combination of platin and taxane alone, for example the cells may have developed resistance to taxane or platin therapy.

The platin or taxane or combination of platin and taxane may be provided in the form of a first composition. The compound of general formula (I) may be provided in the form of a second composition.

In one embodiment the compound of general formula (I) is used in a person who has not yet received, and who is to receive the first composition. In another embodiment, the compound of general formula (I) is used in a person who has received the first composition.

In another embodiment, the compound of general formula (I) is used in a person who has received the first composition. In one embodiment the first and second compositions are used according to a treatment cycle in which:
- the first composition is administered once in a treatment cycle, the first composition being first administered after the second composition is first administered;
- the second composition is administered for at least a period of the treatment cycle.

A treatment cycle commences on the day of administration of the first composition and is completed on the day prior to when a next administration of the first composition would become due. Thus, a first treatment cycle commences on the day of first administration of the first composition and is completed on the day prior to when the second administration of the first composition would become due. The compound of general formula (I) may be used in a person who has received more than one treatment cycle or less than 6 treatment cycles, preferably 2 to 4 treatment cycles. The compound of general formula (I) may be used in a treatment cycle that is greater than 21 to 28 days, preferably 30, 60 or 90 days.

The compound of general formula (I) may be used in the absence of administration of the first composition for a period of time prior to the first treatment cycle.

Preferably the taxane may be paclitaxel and the platin may be carboplatin.

In another embodiment there is provided a use of a compound of general formula (I) as described herein in the manufacture of a medicament for treating or preventing cancer in a person who has received, or is to receive a platin, or a taxane or a combination of a platin and a taxane. In one embodiment the person has received, or is to receive a sub-optimal dose of a platin or a taxane or a combination of a platin and a taxane. The cancer cells may be cells that are not treated by a platin alone, or by a taxane alone, or by a combination of platin and taxane alone, for example the cells may have developed resistance to taxane or platin therapy. Preferably the taxane may be paclitaxel and the platin may be carboplatin.

### Detailed description of the embodiments

Reference will now be made in detail to certain embodiments of the invention. While the invention will be described in conjunction with the embodiments, it will be understood that the intention is not to limit the invention to those embodiments. On the contrary, the invention is intended to cover all alternatives, modifications, and equivalents, which may be included within the scope of the present invention as defined by the claims.

One skilled in the art will recognize many methods and materials similar or equivalent to those described herein, which could be used in the practice of the present invention. The present invention is in no way limited to the methods and materials described.

It will be understood that the invention disclosed and defined in this specification extends to all alternative combinations of two or more of the individual features mentioned or evident from the text. All of these different combinations constitute various alternative aspects of the invention.

As used herein, except where the context requires otherwise, the term "comprise" and variations of the term, such as "comprising", "comprises" and "comprised", are not intended to exclude further additives, components, integers or steps.

In one embodiment there is provided a method for treating or preventing cancer including administering to a person in need thereof:
- a first composition including a platin, a taxane, or combination of same;
- a second composition including a compound of general formula (I) or a pharmaceutically acceptable salt, solvate or polymorph thereof 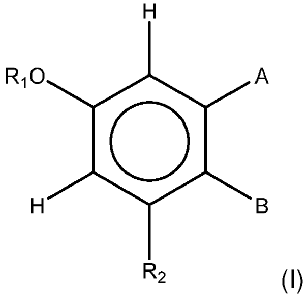 wherein
   R₁ is H, or R_{A}CO where R_{A} is C₁₋₁₀ alkyl or an amino acid;
   R₂ is H, OH, or R_{B} where R_{B} is an amino acid or COR_{A} where R_{A} is as previously defined;
   A and B together with the atoms between them form a six membered ring selected from the group 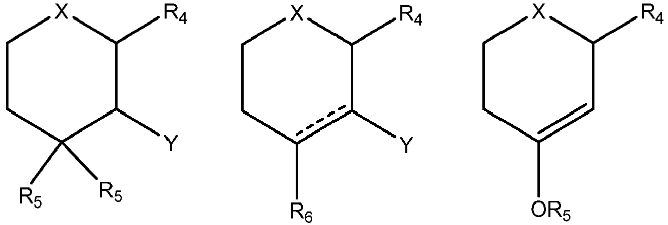 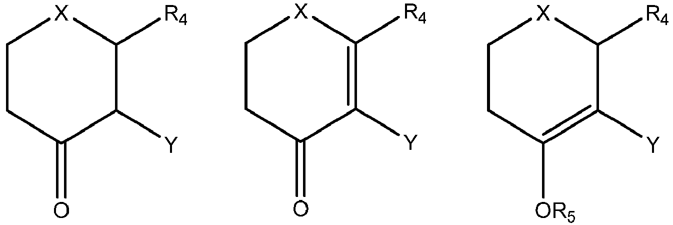 wherein
   R₄ is H, COR_{D} where R_{D} is H, OH, C₁₋₁₀ alkyl or an amino acid, CO₂R_{C} where R_{C} is C₁-₁₀ alkyl, COR_{E} where R_{E} is H, C₁-₁₀ alkyl or an amino acid, COOH, COR_{C} where R_{C} is as previously defined, or CONHR_{E} where R_{E} is as previously defined;
   R₅ is H, CO₂R_{C} where R_{C} is as previously defined, or COR_{C}OR_{E} where R_{C} and R_{E} are as previously defined, and where the two R₅ groups are attached to the same group they are the same or different;
   R₆ is H, CO₂R_{C} where R_{C} is as previously defined, COR_{C}OR_{E} where R_{C} and R_{E} are as previously defined, substituted or unsubstituted aryl or substituted or unsubstituted heteroaryl;
   X is O, N or S;
   Y is 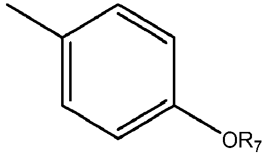 where R₇ is H, or C₁₋₁₀ alkyl; and
   " 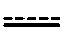 " represents either a single bond or a double bond;
   wherein the second composition is administered to the rectum, vagina or urethra of the person.

In preferred embodiments, the compound of formula (I) is selected from the group consisting of 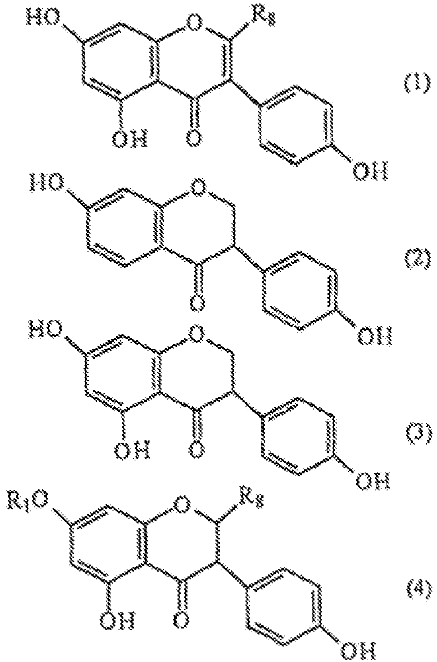 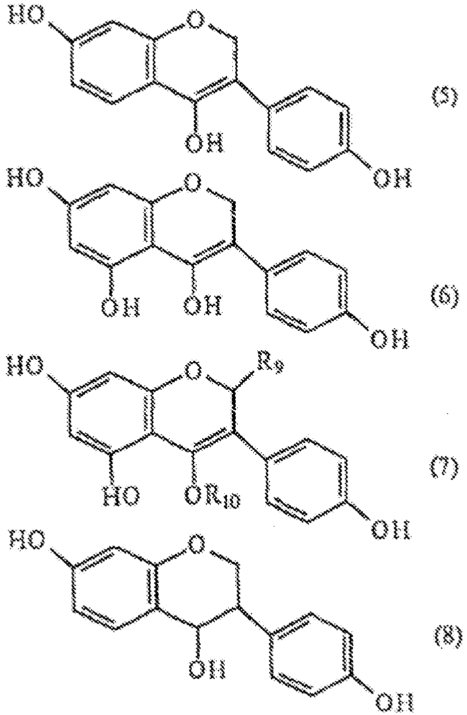 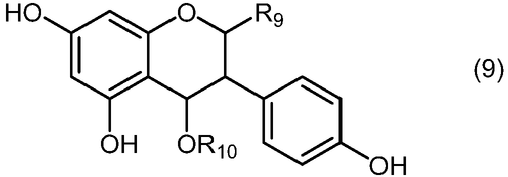 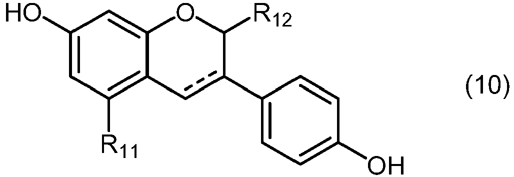 wherein
R₈ is H or COR_{D} where R_{D} is as previously defined;
R₉ CO₂R_{C} or COR_{E} where R_{C} and R_{E} are as previously defined;
R₁₀ is COR_{C} or COR_{C}OR_{E} where R_{C} and R_{E} are as previously defined;
R₁₁ is H or OH;
R₁₂ is H, COOH, CO₂R_{C} where R_{C} and is as previously defined, or CONHR_{E} where R_{E} is as previously defined; and
" 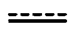 " represents either a single bond or a double bond.

Preferably, the compound of Formula (I) is 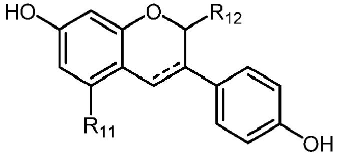 wherein R₁₁ and R₁₂ are as defined above.

Even more preferably, the compound of Formula (I) is 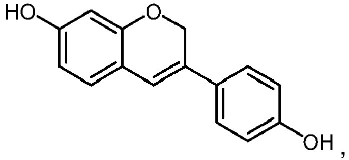 otherwise known as idronoxil (also known as phenoxodiol; dehydroequol; Haginin E (2H-1-Benzopyran-7-0,1,3-(4-hydroxyphenyl)).

In another preferred embodiment, R₆ is substituted or unsubstituted aryl or substituted or unsubstituted heteroaryl. Preferably, R₆ is aryl substituted with an alkoxy group. Preferably, the alkoxy group is methoxy. In another preferred embodiment, R₆ is hydroxy.

In preferred embodiments, the compound of formula (I) may be

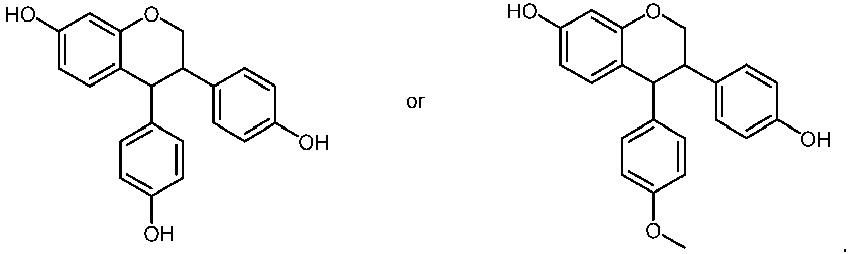

As used herein the term "alkyl" refers to a straight or branched chain hydrocarbon radical having from one to ten carbon atoms, or any range between, i.e. it contains 1, 2, 3, 4, 5, 6, 7, 8, 9 or 10 carbon atoms. The alkyl group is optionally substituted with substituents, multiple degrees of substitution being allowed. Examples of "alkyl" as used herein include, but are not limited to, methyl, ethyl, n-propyl, isopropyl, n-butyl, isobutyl, t-butyl, n-pentyl, isopentyl, and the like.

As used herein, the term "C₁₋₁₀ alkyl" refers to an alkyl group, as defined above, containing at least 1, and at most 10 carbon atoms respectively, or any range in between (e.g. alkyl groups containing 2-5 carbon atoms are also within the range of C₁₋₁₀).

Preferably the alkyl groups contain from 1 to 5 carbons and more preferably are methyl, ethyl or propyl.

As used herein, the term "aryl" refers to an optionally substituted benzene ring. The aryl group is optionally substituted with substituents, multiple degrees of substitution being allowed.

As used herein, the term "heteroaryl" refers to a monocyclic five, six or seven membered aromatic ring containing one or more nitrogen, sulfur, and/or oxygen heteroatoms, where N-oxides and sulfur oxides and dioxides are permissible heteroatom substitutions and may be optionally substituted with up to three members. Examples of "heteroaryl" groups used herein include furanyl, thiophenyl, pyrrolyl, imidazolyl, pyrazolyl, triazolyl, tetrazolyl, thiazolyl, oxazolyl, isoxazolyl, oxadiazolyl, oxopyridyl, thiadiazolyl, isothiazolyl, pyridyl, pyridazyl, pyrazinyl, pyrimidyl and substituted versions thereof.

A "substituent" as used herein, refers to a molecular moiety that is covalently bonded to an atom within a molecule of interest. For example, a "ring substituent" may be a moiety such as a halogen, alkyl group, or other substituent described herein that is covalently bonded to an atom, preferably a carbon or nitrogen atom, that is a ring member. The term "substituted," as used herein, means that any one or more hydrogens on the designated atom is replaced with a selection from the indicated substituents, provided that the designated atom's normal valence is not exceeded, and that the substitution results in a stable compound, i.e., a compound that can be isolated, characterised and tested for biological activity.

The terms "optionally substituted" or "may be substituted" and the like, as used throughout the specification, denotes that the group may or may not be further substituted, with one or more non-hydrogen substituent groups. Suitable chemically viable substituents for a particular functional group will be apparent to those skilled in the art.

Examples of substituents include but are not limited to:
C₁-C₆ alkyl, C₁-C₆ haloalkyl, C₁-C₆ haloalkoxy, C₁-C₆ hydroxyalkyl, C₃-C₇ heterocyclyl, C₃-C₇ cycloalkyl, C₁-C₆ alkoxy, C₁-C₆ alkylsulfanyl, C₁-C₆ alkylsulfenyl, C₁-C₆ alkylsulfonyl, C₁-C₆ alkylsulfonylamino, arylsulfonoamino, alkylcarboxy, alkylcarboxyamide, oxo, hydroxy, mercapto, amino, acyl, carboxy, carbamoyl, aminosulfonyl, acyloxy, alkoxycarbonyl, nitro, cyano or halogen.

The first and second compositions, and active constituents of same are described in more detail below.

### A. First composition

### A.1 Platins

The term "platin" as used herein refers to platinum-based chemotherapeutic agents that are useful in the treatment of proliferative diseases, such as cancers. Some non-limiting examples of platins include cisplatin, oxaliplatin, nedaplatin, lipoplatin, picplatin, satraplatin (JM216), picoplatin (AMD473), nedaplatin, ZD0473, triplatin tetranitrate (BBR3464) and carboplatin, and salts, solvates, polymorphs and derivatives thereof. Preferably, the platin used in the present invention is cisplatin, carboplatin, oxaliplatin or nedaplatin. More preferably, the platin is carboplatin.

The platin concentration to be used in the compositions of the invention may vary based on the carrier(s) co-solubilizer(s), stabilizers, route of administration and on the desired total dose of platin to be administered.

The actual dose to be delivered to a patient varies based on many factors, as a person skilled in the art would appreciate. Doses are often calculated using an estimated creatinine clearance derived from formulas that incorporate the patient's serum creatinine. Other factors include the age, sex, weight, condition of the patient (such as renal impairment), the type and extent of the cancer, whether the patient has been treated previously and others. Various predictors are used to determine drug dosages based on such factors. The Calvert formula is commonly used, which takes into account a patient's glomerular filtration rate (GFR). For carboplatin, the maximum dose is generally capped based on target area under the curve (AUC), and the GFR used in the Calvert formula for carboplatin dosing is recommended not to exceed 125 ml/min.

The Cockcroft-Gault equation is often used to determine drug dosages for patients with impaired renal function, which also has further recommendations for various patients cohorts.

The latest available recommendations together with the health care providers consideration of their individual patients' needs are considered for determining the amount of drug to give to a patient. The recommended dose of carboplatin in previously untreated adults with normal renal function is 400 mg/m² given as a single intravenous infusion over 15 to 60 minutes.

The dosage regime not only includes the amount of drug to be given to the patient but also the frequency of dosing and total number of cycles of medication to administer. These factors will also vary amongst patients and may be determined by several factors as a person skilled in the art can attest, and may be varied based on test results from further monitoring. Administration should not be repeated or repeated at a lesser dose, for example, if the patient's neutrophil or platelet counts are not sufficient.

For example, a typical ovarian cancer patient without renal impairment and being dosed intravenously with carboplatin may be administered the carboplatin every 3 to 4 weeks, for six cycles.

### A.2 Taxanes

The term "taxane" as used herein refers to the group of diterpenes derived from the plants of genus *Taxus* and comprise a taxadiene core and includes taxanes, taxines, and taxoids, as well as derivatives or analogs thereof. The term "taxane" includes both naturally occurring taxanes and taxanes that have been artificially modified or synthesized. Examples of taxanes includes paclitaxel (taxol), docetaxel, carbazitaxel, and salts, solvates, polymorphs and/or derivatives thereof. Preferably, the taxane used in the present invention is paclitaxel or docetaxel. More preferably, the taxane is paclitaxel.

The taxane concentration to be used in the compositions of the invention may vary based on the carrier(s) co-solubilizer(s), stabilizers, route of administration and on the desired total dose of taxane to be administered. Similarly to above for platins, the dosage and dosage regimen of taxanes must also be carefully considered for each patient based on numerous factors. Dosage regimens will also be affected by the response of the patient to treatment, such as severe neutropenia or severe peripheral neuropathy.

For previously treated patients with carcinoma of the ovary, 135-175 mg/m² over 3 hours every 3 weeks is typically used. For breast cancer patients, a typical dose would be 175mg/m² administered intravenously over 3 hours every 3 weeks for 4-6 courses, whilst non-small cell lung cancer would typically be administered intravenously at a dose of either 135mg/m² or 175mg/m² over 3 hours every 3 weeks.

For oral administration, the concentration of taxane in the pharmaceutical compositions may range from about 2 to about 100 mg/ml, preferably from about 6 to about 60 mg/ml or more, preferably from about 10 to about 50 mg/ml.

### A.3 Platin/taxane combinations

Combinations of platin and taxane have proven to be effective in the treatment of some forms of cancer, such as ovarian cancer, including in patients with platinum-resistant cancer. Preferably, the combination of paclitaxel with either carboplatin or cisplatin are utilised. More preferably, the combination of paclitaxel with carboplatin is utilised.

The concentrations of each drug to be given in the compositions of the invention may vary based on the carrier(s) co-solubilizer(s), stabilizers, route of administration and on the desired total dose of platin and taxane to be administered.

Similarly, the treatment regime may vary depending on dose, frequency of administration and type of administration for each of the drugs. For example, when used with cisplatin for the first line treatment of carcinoma of the ovary, paclitaxel is typically used at a concentration of 135mg/m² administered intravenously over 24 hours followed by 75mg/m² cisplatin administered intravenously, or 175mg/m² of paclitaxel administered intravenously over 3 hours followed by cisplatin 75mg/m². Both these treatment regimens are typically repeated every 3 weeks, if, as for the platin drugs, the patient's condition allows for further treatments at these doses.

### A.4 Administration

Platins and taxanes have generally been given intravenously (iv) for treatment of cancer, although there is a growing recognition that these drugs may also be administered by other routes with therapeutic benefit.

Preferably the first composition is adapted for iv administration. Typically the composition is a sterile aqueous preparation of the platin or taxane that is preferably isotonic with the blood of the intended recipient. These preparations may conveniently be prepared by mixing the compound with water or a glycine buffer and rendering the resulting solution sterile and isotonic with the blood.

Injectable formulations generally contain from 0.1% to 60% w/v of platin or taxane and are administered at a rate of 0.1 ml/minute/kg or as appropriate.

Typically the first composition is not given rectally, vaginally or urethrally.

### B. Second composition

### B.1 Isoflavonoids

The term "isoflavonoid" as used herein is to be taken broadly and includes isoflavones, isoflavenes, isoflavans, isoflavanones, isoflavanols and similar or related compounds including those as generally described in formula 1. Some non-limiting examples of isoflavonoid core structures are shown below: 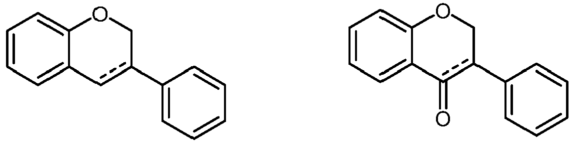 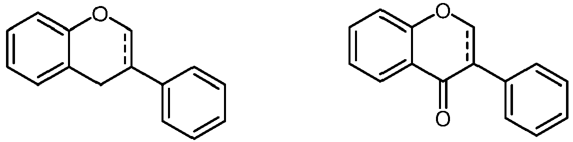 wherein " 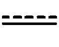 " represents either a single bond or a double bond.

Methods for synthesis of the above described compounds are described in WO1998/008503 and WO2005/049008 and references cited therein towards the synthesis, the contents of which are incorporated herein by reference in entirety.

### B.2 Bases for forming suppository, pessary or urethral devices

In the disclosure below, 'base' may refer to a substance commonly used as a carrier in a suppository, pessary or intra-urethral device.

In one embodiment the base has a solvent power for the compound according to formula (I) enabling at least partial, preferably complete dissolution of the compound according to formula (I) in the base.

The base may be generally oleaginous i.e. hydrophobic or lipohilic.

The base may be comprised of, or consist of an oil or fat.

In one embodiment the base includes saturated fatty acids in an amount of 50 to 65% w/w base. Stearic acid may be included in an amount of 25 to 40% w/w base. Palmitic acid may be included in an amount of 25 to 30% w/w base. Longer chain saturated fatty acids such as myristic, arachidic and lauric acid may be included in an amount of <2% w/w base.

In one embodiment the lipophilic suppository base contains fatty acids and wherein 50 to 100% of the fatty acids of the base are saturated fatty acids, preferably, 90 to 99% of the fatty acids of the base are saturated fatty acids. 30 to 60%, preferably about 40% of fatty acids of the base may be stearic acid. 20 to 30%, preferably about 25% of fatty acids of the base may be palmitic acid. 15 to 25%, preferably about 20% of fatty acids of the base may be lauric acid. 5 to 10%, preferably about 8% of fatty acids of the base may be myristic acid.

Typically, the oleaginous base includes unsaturated fatty acids in an amount of 35 to 50% w/w base. Monounsaturated fatty acid may be included in an amount of 30 to 45% w/w base. Oleic acid may be included in an amount of 30 to 40% w/w base. Polyunsaturated fatty acids such as linoleic and alpha linolenic acid may be included in an amount of 0 to 5% w/w base.

Theobroma oil (cocoa butter) has been a traditional base in a suppository because of: (a) its non-toxic and non-irritant nature, and (b) its low melting point, meaning that it readily dissolves at body temperature when placed within a bodily cavity, However, it is increasingly being replaced for a number of reasons. One reason is its variability in composition, a consequence of its natural origins; theobroma oil also is polymorphic, meaning it has the ability to exist in more than one crystal form. Another is that the formulated product needs to be kept refrigerated because of its low melting point, rendering it unsuitable in tropical regions. This has led to a number of substitute products offering a range of advantages over theobroma oil such as greater consistency, decreased potential for rancidity, and greater ability to tailor phase transitions (melting and solidification) to specific formulation, processing, and storage requirements.

Nevertheless, theobroma oil or a fatty base with similar composition and physicochemical properties has been found to be a preferred embodiment of the invention.

Typically the oleaginous base comprises a predominance of (>45% w/w base) of saturated fatty acids. Preferably the oleaginous base is Theobroma oil (cocoa butter) or an oil fraction or derivative or synthetic version thereof having a saturated fatty acid profile substantially the same as, or identical to the fatty acid profile of Theobroma oil.

Other examples of oils that may be used to provide or obtain fatty acids useful as bases include those obtainable from natural sources such as canola oil, palm oil, soya bean oil, vegetable oil, and castor oil. Oils derived from these sources may be fractionated to obtain oil fractions containing saturated fatty acids.

The base may be formed or derived from a hard fat, butter or tallow.

A base may comprise esterified or non-esterified fatty acid chains. The fatty acid chains may be in the form of mono, di and trigycerides, preferably of saturated fatty acid chains of C9-20 chain length.

A suppository base may be formed from synthetic oils or fats, examples including Fattibase, Wecobee, Witepesoll (Dynamit Nobel, Germany), Suppocire (Gatefosse, France, Hydrokote and Dehydag.

The proportion of the suppository base in the final product is a function of the dosage of active pharmaceutical ingredient and the presence of other pharmaceutical or inert ingredient (if any) but may be provided by way of example in an amount of about 1 to 99% w/w formulation.

### B.3 Manufacture

The suppository, pessary and devices for urethral application of the invention may be prepared as follows. The compound according to formula (I) is contacted with a suppository base (as described above) in molten form in conditions enabling at least partial, preferably complete or substantially complete dissolution of the compound according to formula (I) in the base. This solution is then poured into a suitable mould, such as a PVC, polyethylene, or aluminium mould. For example, the compound according to formula (I) may be contacted with the base at a temperature of from about 35° C to about 50° C and preferably from about 40° C to about 44° C. The compound according to formula (I) can be milled or sieved prior to contact with the base.

In one embodiment, the conditions provided for manufacture, and formulation or device formed from same, enable at least, or provide at least, 50%, preferably 60%, preferably 70%, preferably 80%, preferably 90%, preferably 95% of the compound according to formula (I) for a given dosage unit to be dissolved in the dosage unit. In these embodiments, no more than 50% of the compound according to formula (I) for a given dosage unit, preferably no more than 40%, preferably no more than 30%, preferably no more than 20%, preferably no more than 10%, preferably no more than 5% of compound according to formula (I) for a given dosage unit may be in admixture with, (i.e. undissolved in) the suppository base of the dosage unit.

In one embodiment, all of the compound according to formula (I) added to a dosage unit is dissolved in the base. In this embodiment, no compound according to formula (I) is left in admixture with the suppository base. This is believed to increase the likelihood of the uptake of all of the compound according to formula (I) given in the dosage unit.

It will be understood that the objective of the manufacture process is not to admix, or to mingle, or to blend the suppository base with the compound according to formula (I) as generally occurs in pharmacy practice of admixing components, as it is believed that the resulting admixture would have a lower likelihood of providing therapeutic benefit. In this context, it is particularly important that any other excipient, carrier or other pharmaceutical active does not interfere with the dissolution of the compound according to formula (I) in the base, for example as may occur if the compound according to formula (I) forms a complex with a charged molecular species (other pharmaceutical active, carrier or excipient), the result of which would be to decrease the propensity of the complex, and therefore the compound according to formula (I) contained in it, to dissolve in the suppository base.

Optionally the suppositories, pessaries or intra-urethral devices may be coated, prior to packing, for example with cetyl alcohol, macrogol or polyvinyl alcohol and polysorbates to increase disintegration time or lubrication or to reduce adhesion on storage.

One or more sample suppositories, pessaries, or intra-urethral devices from each batch produced are preferably tested by the dissolution method of the present invention for quality control. According to a preferred embodiment, a sample from each batch is tested to determine whether at least about 75 or 80% by weight of the base dissolves within 2 hours.

Typically the suppository, pessary or like device according to the invention is substantially hydrophobic or lipophilic throughout and does not contain a hydrophilic substance such as hydrophilic carrier or pharmaceutical active, or hydrophilic foci or region formed from the ligation or complexing of the compound according to formula (I) to or with another pharmaceutical compound, carrier or excipient.

Preferably the formulation for forming the suppository, pessary and devices for urethral application does not include a further pharmaceutical active, cytotoxic or chemotherapeutic agent. In this embodiment, the only active in this formulation is the compound according to formula (I) and the formulation does not include a platin, taxane or other cytotoxic or chemotherapeutic agent.

### B.4 Physical characteristics

The total weight of the suppository preferably ranges from about 2250 to about 2700 mg and more preferably from about 2250 to about 2500 mg. According to one embodiment, the suppository has a total weight ranging from about 2300 mg to about 2500 mg.

The suppository or pessary is preferably smooth torpedo-shaped.

The melting point of the suppository or pessary is generally sufficient to melt in the patient's body, and is typically no more than about 37° C.

### C. Cancer treatment

As discussed above, the present invention provides a method for treating or preventing cancer including administering to a person in need thereof a first composition including a platin, a taxane, or combination of same; a second composition including a compound of general formula (I) wherein the second composition is administered to the rectum, vagina or urethra of the person.

The invention recognises that administration of the second composition to the rectum, vagina or urethra of the person enables a practitioner to obtain a desired chemotherapeutic effect with less adverse outcome to the patient. Generally, the shorter each treatment cycle, or the greater the number of treatment cycles required with a conventional therapeutic dose of platin or taxane, the higher the likelihood of adverse outcomes for the patient, be these side effects or drug resistance.

Specifically the invention enables sub optimal doses of platin or taxane to be given; or sub optimal doses of these compounds to be given on a more frequent basis than may be possible for an optimal dose of these compounds; or optimal doses to be given on a less frequent basis, or fewer treatment cycles. These concepts are described further below.

### C.1 Sub optimal doses of platin and/or taxane

In one aspect the method for treating or preventing cancer utilises a sub-optimal or sub-therapeutic dose of a platin, a taxane, or combination of same.

A sub-optimal or sub-therapeutic dose is a dose that is unable to achieve the therapeutic goal. That goal may be for example, a reduction in tumour size, a reduction in increase or decrease of cancer biomarker expression, or mere stasis of tumour growth.

Preferably a sub-optimal dose is one which does not cause significant adverse side effects in the patient.

A sub-optimal dose can be determined according to methods well known in the art. For example, generally an optimal or therapeutic dose for a platin is AUC = 6. A sub optimal dose is generally less than this. This can be further determined by providing an amount of platin less than AUC = 6, and measuring therapeutic impact and/or side effects of platin administration.

In one embodiment, the sub-optimal dose of platin may be AUC of less than 6, preferably from 2 to 5, preferably 3 or 4.

Regarding taxane, an optimal dose is normally expressed as a % of patient body weight. Exemplary ranges are generally from 125-175 mg/m2 for paclitaxel and 60-100 mg/m2 for docetaxel.

In one embodiment, the sub-optimal dose of taxane is 100 mg/m2 for paclitaxel and 50 mg/m2 for docetaxel.

In certain embodiments, the sub-optimal doses may be expressed as a % reduction of a therapeutic or optimal dose. For example, a sub-optimal dose may be provided in 90%, or 80%, or 70%, or 60% or 50%, or 40%, or 30%, or 20%, or 10% of a therapeutic dose.

### C.2 Frequent administration of a sub-optimal dose

Cancer chemotherapy may be administered in treatment cycles. Each treatment cycle typically consists of about 21 to 28 days. For example, in a 21 day treatment cycle, the cycle commences on day 1 which is the day on which the cytotoxic drug is first given. The drug is not given for the following 20 days. The cycle is completed at 20 days from day 1. A second treatment cycle may be provided. This cycle will commence on the day after the 1^{st} treatment cycle is completed by giving the drug on the day after the 1^{st} treatment cycle is completed. The drug is not given for the following 20 days. The second treatment cycle is completed by 20 days from the commencement of the 2^{nd} treatment cycle. Third, fourth, fifth and more treatment cycles may be provided accordingly.

Generally, the reason why a platin or taxane is given once every 21 or 28 days is because the therapeutic dose of the drug is so toxic that the patient cannot receive it more frequently. The sub-therapeutic doses of platin or taxane which are given according to the invention do not cause such significant side effects and therefore are able to be given more frequently than otherwise is possible with a therapeutic dose.

In one embodiment a sub-therapeutic dose of platin and/or taxane in the first composition is given every 7 to 21 days, preferably about every week or 2 weeks.

### C.3. Administration of therapeutic dose less frequently

It is generally understood that the goal of cancer therapy is not achieved where a therapeutic dose is not given at least once a month. The reason for this is that after about 21 to 28 days, the platin or taxane is washed out of the patient.

The therapeutic doses of platin or taxane which are given according to the invention enable an enhanced or potentiated effect of these cytotoxic agents in each treatment cycle, enabling a treatment cycle to be longer than the conventional 21 or 28 day period. This would enable a patient to have more time between outpatient visits for administration of platin or taxane.

In one embodiment, a therapeutic dose is given in a treatment cycle that is greater than 28 days, preferably about 30 to 90 days, preferably about 1.5 to 2 months.

### C.4 Fewer treatment cycles

As mentioned above, cancer treatment regimes are conventionally applied over a 5 to 6 months period, involving about 5 to 6 treatment cycles.

The therapeutic doses of platin or taxane which are given according to the invention enable an enhanced or potentiated effect of these cytotoxic agents in each treatment cycle, thereby requiring few treatment cycles.

In one embodiment a therapeutic dose is given in few than 5 to 6 treatment cycles, preferably 2 to 5 cycles, preferably 3 or 4 cycles.

According to the invention, preferably the first and second compositions are administered according to a treatment cycle in which:
- the first composition is administered after the second composition is first administered;
- the second composition is administered for at least a period of the treatment cycle.

In one embodiment, the second composition is administered daily for a period of at least about 5 days, preferably 5 to 14 days before the first composition is administered.

In one embodiment, the second composition is administered for a period of about 5 to 14 days after the first composition is administered, preferably about 5 to 7 days after the first composition is administered.

In another embodiment, the first composition is first administered at least one day after the day on which the second composition is first administered, although it will be recognised that the first composition may be administered a few days after the second composition is first administered.

The first and second compositions may be given in more than one treatment cycle, preferably less than 5 treatment cycles. Preferably each treatment cycle is less than 21 days where a sub-optimal dose of first composition is given. The treatment cycle may be longer than 28 days where an optimal or therapeutic dose of first composition is given.

In the above described embodiments, preferably the second composition includes idronoxil and the first composition includes carboplatin and/or paclitaxel.

In other embodiments, the administration of the first and second composition may include simultaneous, sequential and/or separate (immediate or prolonged delays between) administration. Sequential and/or separate administration may be in any order. For example, a platin may be administered intravenously prior to or after the rectal, vaginal or urethral administration of a compound of general formula (I) to a person. For another example, a taxane may be administered intravenously prior to or after the rectal, vaginal or urethral administration of a compound of general formula (I) to a person. As a further example, a taxane may be administered intravenously, followed by the intravenous administration of a platin, followed by or preceded by the rectal, vaginal or urethral administration of a compound of formula (I).

As discussed above, it is expected that, when platins and/or taxanes are utilised with the present invention, less platin overall will be needed for treatment regimens.

For example, for the treatment of ovarian cancer patients, it would be expected that platins will be required in quantities from between approximately 50-500 mg/m², preferably in 100-400 mg/m², more preferably in 200-300 mg/m². Cycles will be expected to be from about every 2 to about 5 weeks, preferably every 3-4 weeks, and they will be expected to repeat from about 3 to about 8 cycles, preferably from about 4 to about 6 cycles.

For example, for previously treated patients with carcinoma of the ovary, breast cancer patients or NSCLC patients, it would be expected that taxanes will be required in quantities from between approximately 50-300 mg/m², preferably in 75-175 mg/m². Cycles will be expected to be from about every 2 to about 5 weeks, preferably every 3-4 weeks.

In one embodiment, the second composition is provided according to a variable dosing regimen, wherein a daily amount of the compound according to formula (I) from about 100 to about 3000 mg is provided. In one example, the compound of formula (I) is provided in a dose of about 400mg once daily for a period of 1 to 2 weeks and increased to 800mg once daily for a period of 1 to 2 weeks or 1 month or longer, and further increased to 1600mg (2x800mg) once daily for a period of 1 to 2 weeks or 1 month or longer. Actual amounts will be influenced by disease status, age, weight, gender and other pharmacologically relevant variables.

In one particularly preferred embodiment, the cancer is primary or secondary prostate cancer, the compound of formula (I) is idronoxil and the formulation is in the form of a suppository having a suppository base formed from, or consisting of Theobroma oil (cocoa butter). The idronoxil may be contained in the suppository in an amount of 400mg or 800mg. The idronoxil may be given once or twice daily for a period of 2 to 4 weeks, or for up to 12 months.

Preferably the taxane is paclitaxel and the platin is carboplatin.

In one preferred embodiment, the compound according to formula (I) is idronoxil and it is administered in a quantity of 400mg, 800mg or 1600mg per day over 7-14 days. Paclitaxel is also provided intravenously at a dose of 125mg/m² or 175mg/m² every 21 days and/or carboplatin is provided intravenously at AUC=4 or AUC=5 every 21 days.

It will be understood to the person skilled in the art that for any particular subject, specific dosage regimens should be adjusted according to the individual based on specific circumstances such as severity of disease and the professional judgement of the person directing the administration of the compositions.

Methods for applying a suppository are well known in the art. Generally the methods involve inserting the suppository to a point aligned with the inferior and medial haemorrhoid veins, thereby enabling the release of the drug to the inferior vena cave.

Methods for applying a pessary, or for urethral application of a pharmaceutically active ingredient are well known in the art.

Subjects requiring treatment include those already having a benign, pre-cancerous, or non-metastatic tumor as well as those in which the occurrence or recurrence of cancer is to be prevented.

The objective or outcome of treatment may be to reduce the number of cancer cells; reduce the primary tumor size; inhibit (i.e., slow to some extent and preferably stop) cancer cell infiltration into peripheral organs; inhibit (i.e., slow to some extent and preferably stop) tumor metastasis; inhibit, to some extent, tumor growth; and/or relieve to some extent one or more of the symptoms associated with the disorder.

Efficacy of treatment can be measured by assessing the duration of survival, time to disease progression, the response rates (RR), duration of response, and/or quality of life.

Efficacy of treatment can be measured by assessing the duration of survival, time to disease progression, the response rates (RR), duration of response, and/or quality of life.

In one embodiment, the method is particularly useful for delaying disease progression.

In one embodiment, the method is particularly useful for extending survival of the human, including overall survival as well as progression free survival.

In one embodiment, the method is particularly useful for providing a complete response to therapy whereby all signs of cancer in response to treatment have disappeared. This does not always mean the cancer has been cured.

In one embodiment, the method is particularly useful for providing a partial response to therapy whereby there has been a decrease in the size of one or more tumors or lesions, or in the extent of cancer in the body, in response to treatment.

"Pre -cancerous" or "pre -neoplasia" generally refers to a condition or a growth that typically precedes or develops into a cancer. A "pre -cancerous" growth may have cells that are characterized by abnormal cell cycle regulation, proliferation, or differentiation, which can be determined by markers of cell cycle.

In one embodiment, the cancer is pre-cancerous or pre -neoplastic.

In one embodiment, the cancer is a secondary cancer or metastases. The secondary cancer may be located in any organ or tissue, and particularly those organs or tissues having relatively higher hemodynamic pressures, such as lung, liver, kidney, pancreas, bowel and brain.

Other examples of cancer include blastoma (including medulloblastoma and retinoblastoma), sarcoma (including liposarcoma and synovial cell sarcoma), neuroendocrine tumors (including carcinoid tumors, gastrinoma, and islet cell cancer), mesothelioma, schwannoma (including acoustic neuroma), meningioma, adenocarcinoma, melanoma, leukemia or lymphoid malignancies, lung cancer including small-cell lung cancer (SGLG), non-small cell lung cancer (NSGLG), adenocarcinoma of the lung and squamous carcinoma of the lung, cancer of the peritoneum, hepatocellular cancer, gastric or stomach cancer including gastrointestinal cancer, pancreatic cancer, glioblastoma, ovarian cancer, cervical cancer, liver cancer, bladder cancer, hepatoma, breast cancer (including metastatic breast cancer), colon cancer, rectal cancer, colorectal cancer, salivary gland carcinoma, kidney or renal cancer, prostate cancer, thyroid cancer, hepatic carcinoma, anal carcinoma, penile carcinoma, testicular cancer, esophagael cancer, tumors of the biliary tract, as well as head and neck cancer.

"A condition or symptom associated" [with the cancer] may be any pathology that arises as a consequence of, preceding, or proceeding from the cancer. For example, where the cancer is a skin cancer, the condition or relevant symptom may be microbial infection. Where the cancer is a secondary tumor, the condition or symptom may relate to organ dysfunction of the relevant organ having tumor metastases. In one embodiment, the methods of treatment described herein are for the minimisation or treatment of a condition or symptom in an individual that is associated with a cancer in the individual.

In the above described embodiments, the methods according to the invention may be useful for preventing doubling time of the cancer cells or otherwise inhibiting tumour growth, either through cytotoxic effect on the tumour cells or otherwise by generally inhibiting cell replication.

In another aspect of the invention, the method for treating or preventing cancer is applicable to the treatment of cancer cells that are not treated by a platin alone, or by a taxane alone, or combination of platin and taxane alone.

In another aspect the method for treating or preventing cancer is applicable to the treatment of cancer cells that have acquired resistance to therapy with platin alone, or taxane alone, or combination of platin and taxane alone.

In one embodiment, the treatment provides for an inhibition of increase in PSA score, or for inhibition of tumour growth. In one embodiment the treatment provides for a reduction in PSA score, preferably a 50%, 60%, 70%, 80%, 90% or 100% reduction in PSA score.

### EXAMPLES

### Example 1

NOX66-001 is a Phase I, open-label, non-randomized study. It is a dose-escalation study of dehydroequol delivered rectally in combination with carboplatin in patients with refractory solid tumors who either elect not to have or have failed standard treatment options.

Fifteen patients receive 6 treatment cycles, each of 28-days. Each treatment cycle comprises dehydroequol being administered rectally on Days 1-7 of each cycle. Carboplatin is administered intravenously on Day 2 of each treatment cycle. There will be two different dosages of carboplatin administered (Treatment Regimens 1 and 2), Treatment Regimen 1 being two-thirds the standard dosage of carboplatin (AUC=4) and Treatment Regimen 2 the standard dose (AUC=6). Patients will start with the lower dose of carboplatin and receive that for 3 Treatment Cycles. Providing there is no dose-limiting toxicities, patients then will progress onto the higher dosage of carboplatin which they will receive for up to a further 3 Treatment Cycles.

Patients will be monitored for clinical response (radiographic evidence of tumour numbers and size) and adverse events (toxicity). Of particular interest in this Study is the ability of NOX66 to deliver a meaningful anti-cancer effect in patients with a low dose (AUC = 4) of carboplatin and without significant side-effects.

### Example 2

A patient with late-stage ovarian cancer. Previously treated intravenously with multiple rounds of carboplatin and paclitaxel and now displaying multi-drug resistance by showing disease progression while undergoing combined therapy with carboplatin/paclitaxel. This patient meets the definition of platin- and taxane-refractory disease.

The patient is showing multiple tumours throughout the abdominal cavity and is suffering failing health although still considered sufficiently robust to withstand further cytotoxic chemotherapy were it considered likely to provide a benefit.

The patient is treated with 400 mg of dehydroequol every 12 hours for 7 consecutive days. The dehydroequol is administered rectally in a solid 2 gram suppository dosage form in a theobroma oil base.

Carboplatin is injected intravenously at a dosage equivalent to an AUC of 6 on Day 2 of the 7-day dehydroequol treatment regimen.

This regimen is repeated every 28 days for a total of 6 courses of treatment.

The objective is to induce a response to carboplatin in cancer cells refractory to carboplatin and in so doing prolong the survival of the patient by an additional 12 months.

### Example 3

A patient with castrate-resistant metastatic prostate cancer with secondary cancers in several inguinal lymph nodes and multiple bones (thoracic vertebrae, femur, ribs, humerus). Having progressed on hormone-ablation therapy, the standard next step for this patient is to receive a course of treatment with docetaxel involving an intravenous injection of a dosage of 175 mg/m2 over 3 hours every 21 days for 18 weeks.

Dehydroequol is administered as adjuvant therapy at a dosage of 400 mg once daily for 7 consecutive days commencing on the day prior to the administration of docetaxel and repeated for the duration of the course of docetaxel therapy. The dehydroequol is administered rectally in a solid 2 gram suppository dosage form in a theobroma oil base.

There are two objectives. The first is to increase the response rate from the reported 30% to 40-100%, response being either complete remission, partial remission or no disease progression, resulting in prolonged survival. The second is to increase both the degree and the duration of the response, again feeding into a prolonged survival outcome.

### Example 4

An elderly male patient with non small cell lung cancer with multiple tumours and secondaries to the abdomen and brain and with a survival prognosis of 2 months. The patient has undergone repeated courses of radiotherapy and chemotherapy. The combined effects of the therapy side-effects, advanced age (85 years) and extensive disease state mean that he is considered unsuitable for any further therapy. The patient, nevertheless, requests further treatment.

Dehydroequol is administered as adjuvant therapy in combination with a reduced dosage of carboplatin.

The dehydroequol is given at a dosage of 400 mg twice daily for 7 consecutive days administered rectally in a solid 2 gram suppository dosage form in a theobroma oil base. Treatment is repeated every 28 days for 3 months.

Carboplatin is administered intravenously at a dosage of AUC = 3 on Day 2 of the dehydroequol course of treatment, every 28 days for 3 months.

The objective is to achieve a meaningful anti-cancer effect with shrinkage of all tumours and the development of no new tumours without the occurrence of any treatment side-effects other than slight (Grade 1) tiredness. The expected outcome is a survival benefit of at least 12 months.

### Example 5

A 2-year old child has primary resistant Stage 3 neuroblastoma, meaning that it is not resectable by surgery, has spread locally, and has failed to respond to various previous chemotherapy regimens. The patient is to be treated aggressively with a combination of high dose carboplatin and irinotecan. High dose carboplatin therapy for children normally is in the range 1000 -1200 mg/m2 over a treatment period of 5 days, repeated every 21-28 days on 5-6 occasions. This dosage, while modestly successful in curing the patient, typically results in significant lifelong physical disabilities.

The treatment regimen in this example is combination therapy of carboplatin and irinotecan administered intravenously every 21 days on 5 occasions. The carboplatin dosage is 500 mg/m2 and the irinotecan dosage 50 mg/m2.

Dehydroequol is administered rectally at a dosage of 400 mg daily for 5 consecutive days starting on the day prior to the combined chemotherapy treatment. 400 mg dehydroequol is dissolved in 1 ml of theobroma oil and administered as a liquid rectally (via a tuberculin syringe).

Embodiments of the invention are set out in the following numbered clauses:
1. A method for treating or preventing cancer including administering to a person in need thereof:
   - a first composition including a platin, a taxane, or combination of same;
   - a second composition including a compound of general formula (I) or a pharmaceutically acceptable salt, solvate or polymorph thereof 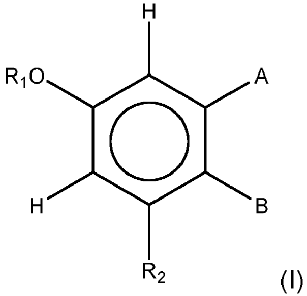 wherein
      R₁ is H, or R_{A}CO where R_{A} is C₁₋₁₀ alkyl or an amino acid;
      R₂ is H, OH, or R_{B} where R_{B} is an amino acid or COR_{A} where R_{A} is as previously defined;
      A and B together with the atoms between them form a six membered ring selected from the group 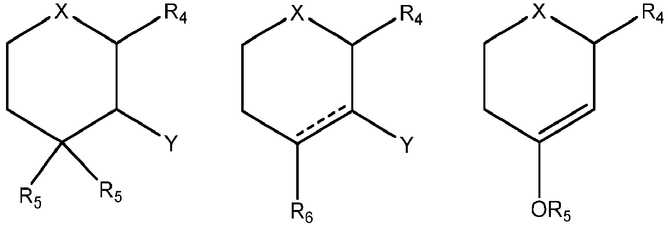 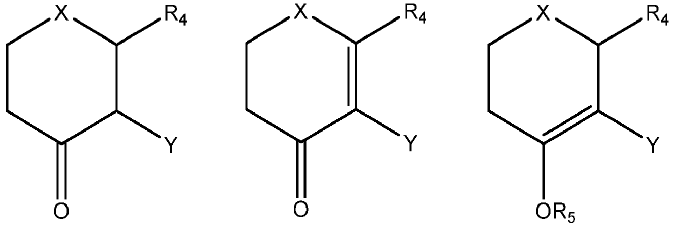 wherein
      R₄ is H, COR_{D} where R_{D} is H, OH, C₁₋₁₀ alkyl or an amino acid, CO₂R_{C} where R_{C} is C₁₋₁₀ alkyl, COR_{E} where R_{E} is H, C₁-₁₀ alkyl or an amino acid, COOH, CORc where Rc is as previously defined, or CONHR_{E} where R_{E} is as previously defined;
      R₅ is H, CO₂R_{C} where R_{C} is as previously defined, or COR_{C}OR_{E} where R_{C} and R_{E} are as previously defined, and where the two R₅ groups are attached to the same group they are the same or different;
      R₆ is H, CO₂R_{C} where R_{C} is as previously defined, COR_{C}OR_{E} where R_{C} and R_{E} are as previously defined, substituted or unsubstituted aryl or substituted or unsubstituted heteroaryl;
      X is O, N or S;
      Y is 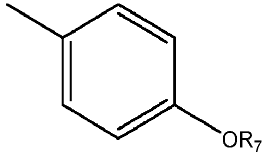 where R₇ is H, or C₁₋₁₀ alkyl; and
      " 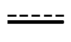 " represents either a single bond or a double bond;
      wherein the second composition is administered to the rectum, vagina or urethra of the person.
2. The method of clause 1, wherein X is O in the compound of general formula (I).
3. The method of clause 1 or clause 1, wherein the compound of general formula (I) is 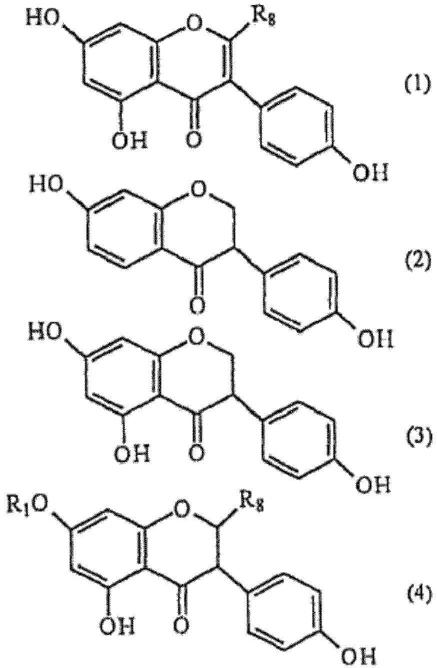 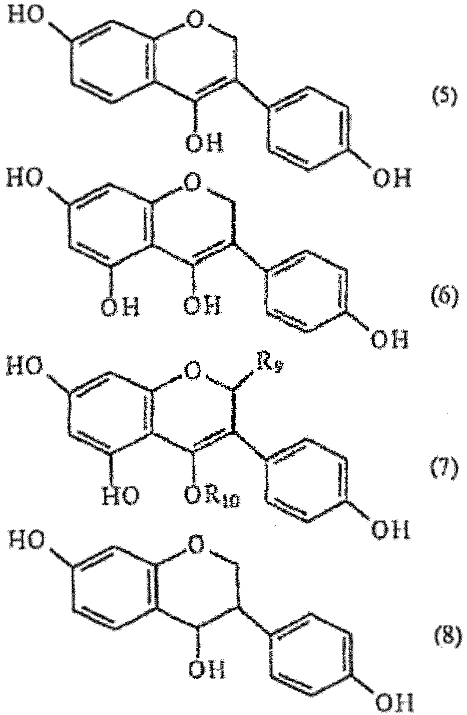 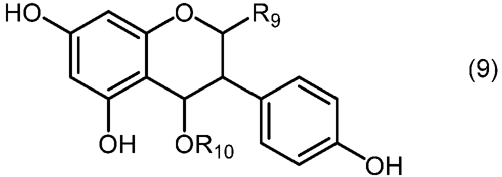 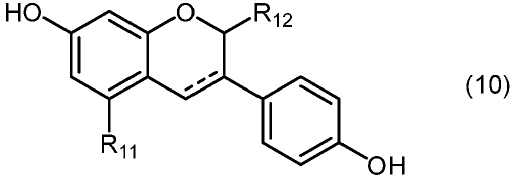 wherein
   R₈ is H or COR_{D} where R_{D} is as previously defined;
   R₉ CO₂R_{C} or COR_{E} where R_{C} and R_{E} are as previously defined;
   R₁₀ is CORc or COR_{C}OR_{E} where Rc and R_{E} are as previously defined;
   R₁₁ is H or OH;
   R₁₂ is H, COOH, CO₂R_{C} where R_{C} and is as previously defined, or CONHR_{E} where R_{E} is as previously defined; and
   " 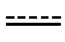 " represents either a single bond or a double bond.
4. The method of any one of the preceding clauses, wherein the compound of general formula (I) is 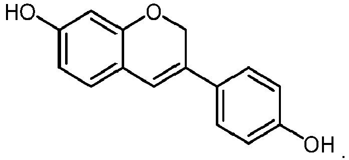
5. The method of clause 1, wherein the compound of general formula (I) is 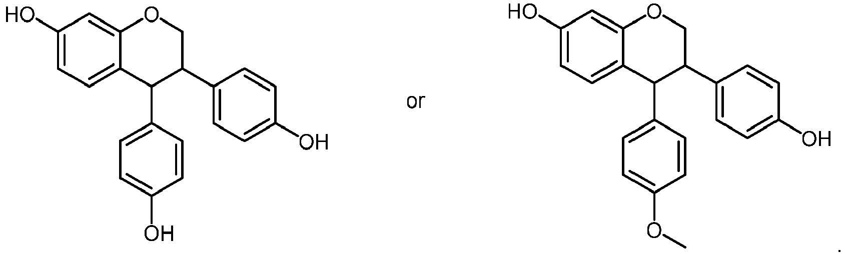
6. The method of any one of the preceding clauses wherein the first composition is administered intra-venously.
7. The method of any one of the preceding clauses wherein the second composition is administered before the first composition is administered.
8. The method of clause 7 wherein the second composition is administered daily for a period of at least about 5 days before the first composition is administered.
9. The method of clause 8 wherein the first composition is administered once every 20 to 30 days.
10. The method of clause 8 wherein the first composition is first administered at least one day after the day on which the second composition is first administered.
11. A method according to any one of clauses 1 to 5, wherein the first and the second composition are co-administered to the rectum, vagina or urethra of the person.
12. A method according to any one of clauses 1 to 5, wherein there is provided the three actives platin, taxane and a compound of formula (I), wherein the platin and the compound of formula (I) are co-administered rectally, vaginally or urethrally and the taxane is administered intravenously.
13. A method according to any one of clauses 1 to 5, wherein there is provided the three actives platin, taxane and a compound of formula (I), wherein the taxane and the compound of formula (I) are co-administered rectally, vaginally or urethrally and the platin is administered intravenously.
14. The method of any one of clauses 1 to 5 wherein the first and second compositions are administered according to a treatment cycle in which:
   - the second composition is administered daily for a period of no more than about 14 days;
   - the first composition is administered once in the treatment cycle, the first composition being administered on the day after the first day on which the second composition is administered.
15. The method of clause 14 wherein the treatment cycle consists of no more than 30 days.
16. The method of clause 15 wherein the treatment cycle consists of about 21 days.
17. The method of any one of clauses 1 to 5 wherein the second composition is administered so as to provide a daily amount of the compound according to formula (I) of from about 100 to about 3000 mg.
18. The method of clause 17, wherein the second composition is administered so as to provide a daily amount of the compound according to formula (I) of from about 400 to about 1600 mg.
19. The method of any one of the preceding clauses, wherein the first composition comprises a platin in an amount of from about 50-500 mg/m².
20. The method of clause 19 wherein the first composition comprises a platin in an amount of from about 100-400 mg/m².
21. The method of clause 20, wherein the first composition comprises a platin in an amount of from about 200-300 mg/m².
22. The method of any of the preceding clauses, wherein the first composition comprises a taxane in an amount of from about 50-300 mg/m².
23. The method of clause 22, wherein the first composition comprises a taxane in an amount of from about 75-175 mg/m².
24. The method of any one of the preceding clauses wherein the second composition is administered by the person in need of cancer treatment or prevention.
25. The method of any of the preceding clauses, wherein the taxane is paclitaxel.
26. The method of any of the preceding clauses, wherein the platin is cisplatin, carboplatin, oxaliplatin or nedaplatin.
27. The method of clause 25, wherein the platin is carboplatin.
28. The method of any of the preceding clauses, wherein the second composition is administered rectally.

## Claims

1. A first composition including a platin, a taxane, or combination of same for use in a method of treating or preventing cancer in a person in need thereof, wherein the cancer is selected from the group consisting of prostate cancer, breast cancer, ovarian cancer, non-small cell lung cancer, neuroblastoma and sarcoma, wherein the treatment or prevention includes administering:
- a second composition including idronoxil represented by the following structural formula: 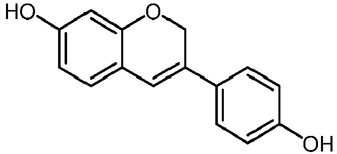
or a pharmaceutically acceptable salt, solvate or polymorph thereof,
wherein a therapeutic dose of the first composition is administered in a treatment cycle that is greater than 28 days;
wherein the first composition is administered intra-venously, and wherein the second composition is administered to the rectum, vagina or urethra of the person.

2. A second composition including idronoxil represented by the following structural formula: 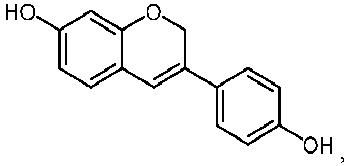
or a pharmaceutically acceptable salt, solvate or polymorph thereof, for use in a method of treating or preventing cancer in a person in need thereof,
wherein the cancer is selected from the group consisting of prostate cancer, breast cancer, ovarian cancer, non-small cell lung cancer, neuroblastoma and sarcoma,
wherein the treatment or prevention includes administering a first composition including a platin, a taxane or combination of the same,
wherein a therapeutic dose of the first composition is administered in a treatment cycle that is greater than 28 days;
wherein the first composition is administered intra-venously, and wherein the second composition is administered to the rectum, vagina or urethra of the person.

3. The composition for use of claim 1 or 2, wherein the second composition is administered before the first composition is administered.

4. The composition for use of claim 3, wherein the second composition is administered daily for a period of at least 5 days before the first composition is administered.

5. The composition for use of claim 4, wherein the first composition is first administered at least one day after the day on which the second composition is first administered.

6. The composition for use of any one of claims 1 to 3, wherein the first and second compositions are administered according to a treatment cycle in which:
- the second composition is administered daily for a period of no more than 14 days;
- the first composition is administered once in the treatment cycle, the first composition being administered on the day after the first day on which the second composition is administered.

7. The composition for use of any one of the preceding claims, wherein the treatment cycle is 30 to 90 days, preferably 1.5 to 2 months.

8. The composition for use of any one of the preceding claims, wherein the first composition is administered in fewer than 5 to 6 treatment cycles, preferably 2 to 5 cycles, more preferably 3 or 4 cycles.

9. The composition for use of any one of the preceding claims, wherein the second composition is administered so as to provide a daily amount of idronoxil of from 100 to 3000 mg, or from 400 to 1600 mg.

10. The composition for use of any one of the preceding claims, wherein the taxane is paclitaxel, docetaxel, or carbazitaxel.

11. The composition for use of any one of the preceding claims, wherein the platin is cisplatin, carboplatin, oxaliplatin or nedaplatin, preferably carboplatin.

12. The composition for use of any one of the preceding claims, wherein the idronoxil is at least partially dissolved in an oleaginous base.
